# EUROPEAN PATENT APPLICATION

(11) **EP 3 127 536 A1**
(43) Date of publication of application: **08.02.2017**
(21) Application number: 16182805.8
(22) Date of filing: 04.08.2016
(51) Int. Cl.: A61K 9/00, A61K 9/20, A61K 31/00

(54) **MELATONIN FORMULATIONS AND METHODS FOR PREPARATION AND USE**

(30) Priority: 05.08.2015 NL 2015272
(71) Applicant: Versailles B.V., 1112 AX Diemen (NL)
(72) Inventor: De Naeyer, An, 8500 Kortrijk (BE); van den Berg, Johannes Dirk, 1112 AX Diemen (NL)
(74) Representative: Brantsandpatents bvba

(57) **Abstract**

The current invention concerns a method for manufacturing formulation solid formulation comprising melatonin and a tablet produced from such solid formulation, as well as the medical use thereof.

## Description

### TECHNICAL FIELD

The invention pertains to the technical field of melatonin formulations and methods for preparation and use of the latter

### BACKGROUND

Hormones such as the indole hormone, melatonin, are widely distributed in both plant and animal sources. Melatonin can be found in human milk, bananas, beets, cucumbers, and tomatoes. Chemically, melatonin is N-acetyl-5-methoxytryptamine, a derivative of serotonin, which in turn is derived from tryptophan. Melatonin is a ubiquitous natural neurotransmitter-like compound produced primarily by the pineal gland, and is involved in numerous aspects of the biological and physiologic regulation of body functions. The role of endogenous melatonin in circadian rhythm disturbances and sleep disorders is well established. Some studies have shown that melatonin may also be involved in breast cancer, fibrocystic breast diseases, and colon cancer. Melatonin has been shown to modify immunity, the stress response, and certain aspects of the aging process. Some studies have demonstrated improvements in sleep disturbances and "sundowning" in patients diagnosed with Alzheimer's disease. The antioxidant role of melatonin may be of potential benefit for conditions in which oxidative stress is involved in the pathophysiologic processes. The multiplicity of actions and variety of biological effects of melatonin suggest the potential for a range of clinical and wellness-enhancing uses, especially considering that as one ages, the production of this key hormone goes into steady decline. Indeed, for an octogenarian, the amount produced is quite nominal.

Through melatonin release, the pineal gland maintains the internal clock governing the natural rhythms of body function. This apparent clock-setting property of melatonin has led to the suggestion that it is a "chronobiotic" substance that alters and potentially normalizes biological rhythms and adjusts the timing of other critical processes and biomolecules (hormones, neurotransmitters, etc.) that, in turn, exert numerous peripheral actions. The sleep-inducing effects of melatonin have advantages over conventional hypnotics, since melatonin, itself, is not a hypnotic drug. Melatonin only induces a natural state of sleepiness, and does not have the adverse side-effects of conventional hypnotics and prescription sleeping aids.

Melatonin has previously been used pharmaceutically, and has been prepared for oral administration (see, e.g., WO 1995/003043). These preparations include melatonin formulated with a cyclodextrin (WO 1999/047175), and as a microemulsion (US Patent No. 5,362,745).

However, the manufacturing of an oral formulation conforming to ICH pharmaceutical guidelines comprising melatonin has been proven difficult (e.g. tablet), which could be due to the intrinsic properties of melatonin. Melatonin exhibits a high degree of agglutination and adhesiveness, which hampers the manufacturing of a homogenous batch of tablets. Moreover, melatonin is poorly soluble in water, which may affect its bioavailability and the release of melatonin in the body.

Many of the conventional manufacturing methods for solid formulations known in the art fail to deliver formulations with a good uniformity of dosage units results. As such, the formulations are not in conformity with the regulatory requirements.

Accordingly, there is a need in the medical and pharmaceutical arts to provide an oral dosage form, preferably as an immediate release formulation, compliant with uniformity of dosage units requirements. This need is met by the method of preparation according to the current invention.

### SUMMARY OF THE INVENTION

The present invention provides for a method for the manufacturing of a solid formulation according to claim 1. The methodology ensures good uniformity of dosage units results, which is a key requirement according to the pharmaceutical guidelines. In a second aspect, the current invention provides a tablet according to claim 10.

In particular, the current invention is characterized by the following embodiments, which should not be construed as limiting.
1. A method for the manufacturing of a solid formulation comprising melatonin as active pharmaceutical ingredient, said method comprises a melatonin dispersion step in one or more intragranular excipients to form a melatonin blend, followed by a wet granulation step.
2. Method according to embodiment 1, whereby said intragranular excipients in the dispersion step are chosen from fillers, binders, disintegrants, and/or glidants.
3. Method according to any one of the embodiments 1 or 2, characterized in that a binder solution is granulated with said melatonin blend.
4. Method according to embodiment 3, characterized in that said wet granulation occurs by means of a high shear granulator, fluidized bed granulator, twin screw granulator.
5. Method according to any one of the previous embodiments, characterized in that said dispersion step comprises the formation of a premix, said premix comprises melatonin and one or more intragranular excipients, followed by a sieving step of said premix and further blending with one or more intragranular excipients to form said melatonin blend.
6. Method according to embodiment 5, characterized in that said sieving is performed by use of a mesh with mesh size between 800 and 1200 micron.
7. Method according to embodiment 6, comprising the following sequence of steps:
   - dispersion of melatonin with one or more fillers and a glidant;
   - providing a binder solution;
   - wet granulation of said melatonin blend with said binder solution;
   - drying the granulate;
   - addition of a lubricant to said granulate; and mixing of the final blend.
8. Method according to embodiment 7, whereby said final blend is tableted.
9. Method according to embodiment 7 or 8, characterized in that said ratio between melatonin, glidant and filler is between 1:0.1:4 and 1:0.3:6.
10. Method according to any one of the previous embodiments, characterized in that said melatonin has a particle size distribution determined by laser-diffraction or comparable method as follows:
   - d(0.1) = 10 µm
   - d(0.5) = 50 µm
   - d(0.9) = 210 µm.
11. Tablet comprising melatonin, whereby said tablet is an immediate release formulation and whereby said tablet comprises:
   - between 0.5 and 10% w/w of melatonin;
   - between 75 and 99% w/w of one or more fillers;
   - between 0.5 and 5% w/w of one or more binders;
   - between 0.05 and 0.5% of one or more glidants; and
   - between 0.25 and 1% w/w of one or more lubricants.
12. Tablet according to embodiment 11, characterized in that the formulation for the tablet is obtained via the method according to any of the embodiments 1 to 10.
13.Tablet according to any one of the previous embodiments, characterized in that said filler is selected from cellulose microcrystalline, lactose monohydrate, sucrose, glucose, polyols, calcium carbonate, and/or magnesium stearate, mixtures or derivatives thereof.
14. Tablet according to any one of the previous embodiments, whereby said binder is selected from cellulose or modified cellulose such as hydroxycellulose, microcrystalline cellulose; polyethylene glycol; polyvinylpyrrolidone; starch and/or mixtures thereof.
15. Tablet according to any one of the previous embodiments, whereby said glidant is selected from (colloidal) silicon dioxide, starch, talc, magnesium stearate and/or a mixture thereof.
16. Tablet according to any one of the previous embodiments, comprising particulate melatonin, characterized in that said melatonin has a particle size distribution profile as follows:
   - d(0.1) = 10 µm
   - d(0.5) = 50 µm
   - d(0.9) = 210 µm.
17. Tablet according to any one of the previous embodiments, for use in the treatment of sleep problems chosen from delayed sleep phase disorders, primary insomnia for adults aged 55+ and general sleep disorders and treatment of difficulties in sleep initiation and maintenance that are not associated with a medical condition, substance use or concurrent psychological disorder.
18. Tablet according to any one of the previous embodiments 11 to 16, for use in the treatment of ADHD, concentration and/or attention problems and/or hyperactivity.

### DESCRIPTION OF FIGURES

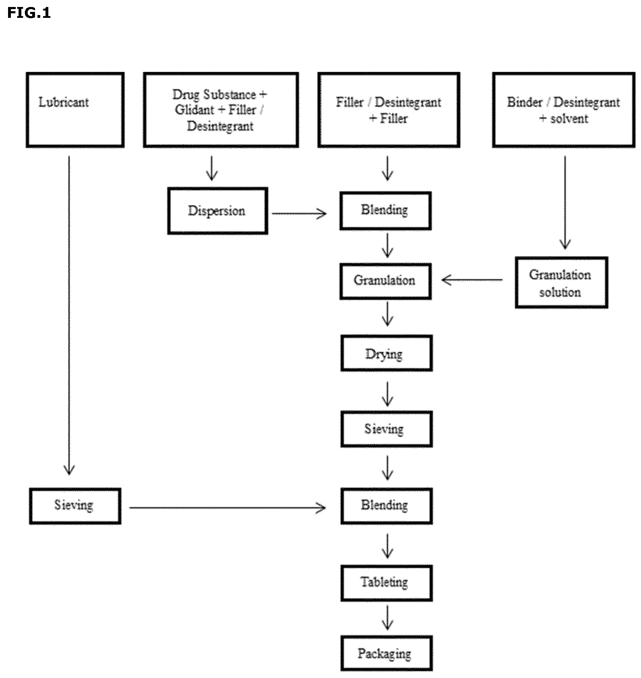

**Figure 1** shows a flowchart of a methodology according to the current invention.

### DETAILED DESCRIPTION OF THE INVENTION

The present invention concerns a solid formulation comprising physically and chemically defined melatonin and a method for production of a solid pharmaceutical formulation. This combination ensures a homogeneous distribution of the melatonin throughout the formulation, a fast disintegration of the tablet and optimal bioavailability of the melatonin.

Melatonin was found to exhibit a high degree of adhesiveness or stickiness when being used in formulations. Moreover, melatonin has a low solubility in aqueous media, therefore solubility/dissolution can potentially be a source of variable absorption with an impact on its pharmacokinetic profile. When administered orally, melatonin shows low and variable bioavailability. With a reported weak basic pKa function of approximately 4.4 to 4.7, melatonin will have different degrees of dissociation and different solubility as it travels through the gastro-intestinal tract. Solubility being higher in the stomach and decreasing throughout the intestinal tract as pH increases. Although permeability on the other hand is generally good, it also appears site dependent being very low in the stomach and in increasing order in ileum < jejunum < colon (Tran et al., 2009). Therefore, the amount of melatonin available for absorption into the bloodstream primarily depends on its solubility and the compound has been classified in both class I and class II category according to the Biopharmaceutics Classification System without a common consensus.

These properties influence the design / development of pharmaceutical tablets comprising melatonin. Especially, it was found that the uniformity of dosage units, which relates to the consistency of dosage units and which is a requirement for being accepted as a pharmaceutical device, was influenced.

The inventors of the current invention have now found that by using a specific production process, a solid formulation which may be used for tablets with a good uniformity of dosage units and pharmacokinetic profile can be obtained.

In the context of the current invention, the term "pharmaceutical" is to be understood as a formulation or composition which is in conformity with the ICH and applicable EU requirements for qualification as a medicament to be authorized by a competent and legally qualified organisation.

In the context of the current invention, the term "dosage units" is to be understood as dosage forms containing a single dose or a part of a dose of drug substance in each unit.

In the context of the current invention, the term "Uniformity of dosage units" is to be understood as the degree of uniformity in the amount of the drug substance among dosage units.

In the context of the current invention, the term "particle size distribution" (PSD) is to be understood as an index (means of expression) indicating what sizes (particle size) of particles are present in what proportions (relative particle amount as a percentage where the total amount of particles is 100 %) in the sample particle group to be measured. Said PSD is defined on the basis of individual points on the cumulative distribution curve, represented as d(0.X) = Y (where X and Y are Arabic numerals), each "d" describing an individual point on the cumulative PSD curve. The number "X" represents the percentage (number, volume, or weight) of particles in the population having a nominal size up to and including "Y". That is, d(0.9) = 41µm is the distribution such that 90% of the particles have a width of this value or less. When 41µm < d(0.9) ≤81 µm, it means that 90% of the particles conform to a value for width within this range.

The expression "% by weight" or "w/w" (weight percent), here and throughout the description unless otherwise defined, refers to the relative weight of the respective component based on the overall weight of the formulation.

The term "excipient" is to be understood as a natural or synthetic substance formulated alongside the active ingredient of formulation included for the purpose of bulking-up formulations that contain active ingredients or to confer a therapeutic enhancement on the active ingredient in the final dosage form, such as facilitating drug absorption or solubility, or to aid in the manufacturing of the formulation.

The term "glidant" is to be understood as a substance which is added to a solid formulation to improve its flowability.

The term "binder" is to be understood as a substance which holds ingredients together, ensuring that tablets and granules can be formed.

The term "filler" or "diluent" is to be understood as a substance which is a diluting agent or for providing substance to a formulation. A filler is generally inactive.

The term "lubricant" is to be understood as a substance which prevents the finished product from adhering to the surface of the tableting equipment.

The term "disintegrant" is to be understood as a substance which facilitates the disintegration of the pharmaceutical product into smaller particles upon exposure to changed environment, e.g. solvent.

The term "intragranular" is used to indicate the constituents of granules formed during a granulation process.

In a first aspect, the current invention provides a method for the manufacturing of a solid formulation comprising melatonin as an active pharmaceutical ingredient. In particular, said method comprises a melatonin dispersion step in one or more intragranular excipients to form a melatonin blend, followed by a wet granulation step. It was found by the inventors of the current invention that this two-step sequence allows the production of a melatonin formulation with excellent uniformity of dosage units and good disintegration characteristics. Other commonly known methodologies such as direct compression based methodologies or direct wet granulation were found to be inadequate for reaching these goals.

Said intragranular excipients in the dispersion step are preferably chosen from fillers, binders, disintegrants, and/or glidants. In an embodiment, said melatonin blend is comprised of one or more intragranular excipients as listed above. In another embodiment, said melatonin blend is comprised of all intragranular excipients as listed afore.

In a preferred embodiment, a binder solution will be granulated with said melatonin blend in a wet granulation step. To that purpose, a suitable binder will be mixed with a solvent, such as (purified) water or acetone. The binder solution is subsequently added to a melatonin blend (preferably not comprising any additional binder). The wet granulation preferably occurs by means known in the art such as a fluidized bed granulator, a twin screw granulator or a high shear granulator. In a preferred embodiment, said wet granulation occurs in a high shear granulator. As mentioned, the solvent used during wet granulation is preferably water or an organic solvent such as aceton. By preference, water is used as solvent, as organic solvents may be hazardous during production.

In a preferred embodiment, the melatonin blend is achieved via a two-step dispersion. First, a premix is formed. Such premix comprises melatonin and one or more intragranular excipients. Suitable excipients are e.g. one or more fillers and/or a glidant. The premix is formed by blending of a suitable amount of ingredients. Subsequently, said premix is sieved to attribute to favorable compression characteristics. Finally, said premix is further blended with one or more intragranular excipients. It will be understood for a skilled person that such premix may comprise of various suitable intragranular excipients. For instance, a possible premix may comprise of melatonin and a filler. In another embodiment, said premix may comprise of melatonin, one or more fillers and a glidant or a disintegrant. It will also be understood for a skilled person that the premix may comprise only a subamount of the total amount of a certain intragranular excipient to be used in the formulation. Any remaining parts may be added after sieving to form the melatonin blend.

It was found by the inventors of the current invention that use of a premix comprising the API has a beneficial effect on the dispersion of the API in the final product.

In one aspect of the invention, the ratio between melatonin and intragranular excipients in the premix will be between 1:2 and 1:8, more preferably between 1:4 and 1:6. In a further, more preferred embodiment, the extragranular excipients comprise a filler and a glidant/disintegrant, whereby the ratio between melatonin, glidant or disintegrant and filler is between 1:0.1:4 and 1:0.3:6, more preferably 1:0.2:5.

The premix is preferably sieved in order to remove big particulates. By preference, sieving will occur with a sieve having a mesh size between 600 and 1200 micron, more preferably between 800 and 1200 micron.

After granulation, the obtained granules are dried, e.g. by means of a fluid bed granulator and optionally sieved again to remove unwanted big particulates. Subsequently, further extragranular excipients may be added to the granules. In a preferred embodiment, a lubricant is added. In another or further embodiment, artificial sweeteners or flavors can be added. The extragranular excipients are blended with the granules, e.g. in a Y-cone mixer.

A preferred embodiment of the current invention comprises the following steps:
- dispersion of melatonin with one or more fillers and a glidant;
- providing a granulation or binder solution, said solution comprising a binder;
- wet granulation of said melatonin blend with said solution;
- drying the granulate;
- addition of a lubricant to said granulate; and
- mixing of the final blend.

The dosage form of the invention may be a capsule containing the formulation, preferably a powdered or granulated solid composition of the invention. The shell may be made from gelatin or hydroxypropylmethylcellulose and optionally contain a plasticizer such as glycerin and sorbitol, and an opacifying agent and/or colorant.

In a preferred embodiment, the active ingredient according to the current invention is formulated in a tablet, e.g. a tablet of between 50 and 200 mg, preferably 100 to 110 mg.

Finally, the formulation is tableted. Tableting occurs preferably by use of a compression force which is between 10 kN and 15 kN. It was found that the latter range does not influence the dissolution profile.

The melatonin used in the current invention preferably has a well-defined particle size distribution determined by laser-diffraction or comparable method. In an embodiment of the current invention, said melatonin has a particle size distribution (PSD) of d(0.9)> 210 µm as determined by laser-diffraction method. In a further preferred embodiment, said PSD of melatonin is within the following range: d(0.9)= about 60 µm to 210 µm.

In another or further embodiment, said melatonin has a particle size distribution of d(0.5)> 50 µm. In a more preferred embodiment, said PSD of melatonin is within the following range: d(0.5) = about 5 µm to 50 µm.

In yet another or further embodiment, said melatonin has a particle size distribution of d(0.1)> 10 µm. In a further preferred embodiment, said PSD of melatonin is within the following range: d(0.1) = about 0.5 µm to 10 µm.

Preferably, the melatonin in the formulation according to the current invention has a particle size distribution profile as follows:
- d(0.1) = about 0.5 µm to 10 µm
- d(0.5) = about 5 µm to 50 µm
- d(0.9) = about 60 µm to 210 µm.

The PSD of the solid particulate melatonin of the invention is preferably determined using the well-known laser diffraction method. The laser-diffraction method (low angle laser light scattering) is sensitive to the volume of a particle and provides a volume-average particle size, which is equivalent to the weight-average particle size if the density is constant. In some instances, particle size determination by laser diffraction employs the so-called Fraunhofer approximation - based on projected area - which includes the assumptions that particles size is not smaller than the wavelength of light constant scattering efficiency, and particle opacity. The "Mastersizer S" from Malvern Instruments equipped with a small cell dispersion unit is an example of a laser-diffraction particle size analyzer useful in determining the PSD of solid particulate melatonin of the present invention. However, any laser diffraction method that will give an accurate result can be used.

The small and large particle size solid particulate melatonin can be prepared by conventional techniques. For example, after being characterized for size in its raw state, the melatonin may be milled, for example using a pin mill under suitable conditions of mill rotation rate and feed rate, to bring the particle size value within a desirable range. The efficiency of the milling may be monitored by sampling. If a first pass through the mill fails to produce the required size distribution, then one or more further passes are effected. Other milling methods are also suitable, including, a variety of milling techniques, such as hammer or fluid energy mills.

It is postulated that a more homogenous distribution of melatonin in the solid formulation is achieved, which positively affects the uniformity of dosage units and possibly also the bioavailability of the melatonin.

In a second aspect, the current invention is directed to a tablet comprising melatonin. The tablet may be a slow- or prolonged release or immediate release formulation. For the purpose of the current invention, said prolonged release is to be understood as a formulation which only gradually releases its active ingredient in the bloodstream of a patient, e.g. over a period of 4 to 12 hours.
Preferably, said tablet is an immediate release formulation. Said immediate release is to be understood as a formulation which releases the active ingredient in a time period of no more than 0.5 hour, occurring in the blood stream of the patient, in typically less than 60 minutes. It was found by the inventors of the current invention that an immediate release formulation more closely mimics the naturally occurring kinetics of endogenous melatonin, e.g. when used for sleep problems.

The tablet according to the current invention has preferably the following composition:
- between 0.5 and 10% w/w of melatonin;
- between 75 and 99% w/w of one or more fillers;
- between 0.5 and 5% w/w of one or more binders;
- between 0.05 and 0.5% of one or more glidants; and
- between 0.25 and 1% w/w of one or more lubricants.

The inventors of the current invention found that the latter composition is therapeutically effective, provides a good bio-availability and provides a good uniformity of dosage units results, necessary for pharmaceutical compositions. Hence, the composition is in conformity with the relevant regulatory requirements. By preference, the tablet is obtained via the method as described above.

A unit dose may comprise between 0.1 and 10 mg, more preferably between 0.5 and 7.5 mg melatonin, more preferably between 1 and 5 mg, such as 1, 3 or 5 mg of melatonin.

As mentioned above, the particulate melatonin preferably has a specific particle size distribution. It is postulated by the inventors that such particle size distribution has a positive influence on the uniformity of dosage units results, and the bio-availability of melatonin.

By preference, said melatonin has a particle size distribution (PSD) in said tablet of d(0.9)> 210 µm as determined by laser-diffraction method. In a further preferred embodiment, said PSD of melatonin is within the following range: d(0.9)= about 60 µm to 210 µm.

In another or further embodiment, said melatonin has a particle size distribution of d(0.5)> 50 µm. In a more preferred embodiment, said PSD of melatonin is within the following range: d(0.5) = about 5 µm to 50 µm.

In yet another or further embodiment, said melatonin has a particle size distribution of d(0.1)> 10 µm. In a further preferred embodiment, said PSD of melatonin is within the following range: d(0.1) = about 0.5 µm to 10 µm.

Preferably, the melatonin in the tablet according to the current invention has a particle size distribution profile as follows:
- d(0.1) = about 0.5 µm to 10 µm
- d(0.5) = about 5 µm to 50 µm
- d(0.9) = about 60 µm to 210 µm.

In addition to the active ingredient(s), in the current case melatonin, the formulation of the invention contains one or more excipients. Excipients are added to the formulation for a variety of purposes. By preference, said excipients - are chosen from fillers, binders, disintegrants, sweeteners, coatings, lubricants and/or glidants.

Diluents or fillers increase the bulk of a solid pharmaceutical composition, and may make a pharmaceutical dosage form containing the composition easier for the patient and care giver to handle. Diluents suitable for tablets according to the current invention include, for example, cellulose (e.g. Avicel®), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit®), potassium chloride, powdered cellulose, sodium chloride, sorbitol and talc.

In a preferred embodiment, said filler is selected from cellulose microcrystalline, lactose monohydrate, sucrose, glucose, polyols, calcium carbonate, and/or magnesium stearate, mixtures or derivatives thereof.

Solid formulations that are compacted into a dosage form, such as a tablet, may include excipients whose functions include helping to contain the active ingredient-and other excipients together after compression. Suitable binders include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel®), hydroxypropyl methyl cellulose (e.g. Methocel®), liquid glucose, magnesium aluminum silicate, maltodextrin, methylcellulose, polymethacrylates, povidone (e.g. Kollidon®, Plasdone®, pregelatinized starch, sodium alginate and starch.

In a preferred embodiment, said binder is selected from cellulose or modified cellulose such as hydroxycellulose, microcrystalline cellulose; polyethylene glycol; polyvinylpyrrolidone; starch and/or mixtures thereof.

The dissolution rate of a compacted solid pharmaceutical composition in the patient's stomach may be increased by the addition of a disintegrant to the composition. Suitable disintegrants include alginic acid, carboxymethylcellulose calcium, carboxymethylcellulose sodium (e.g. Ac-Di-Sol®, Primellose®), colloidal silicon dioxide, croscarmellose sodium, crospovidone (e.g. Kollidon®, Polyplasdone®), guar gum, magnesium aluminum silicate, methyl cellulose, microcrystalline cellulose, polacrilin potassium, powdered cellulose, pregelatinized starch, sodium alginate, sodium starch glycolate (e.g. Explotab®) and starch.

Glidants can be added to improve the flowability of a non-compacted solid composition and to improve the accuracy of dosing. Excipients that may function as glidants in the current invention include colloidal silicon dioxide, magnesium trisilicate, magnesium stearate, powdered cellulose, starch, talc and tribasic calcium phosphate.

In a preferred embodiment, said glidant is selected from (colloidal) silicon dioxide, starch or talc.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl furmarate, stearic acid, talc and zinc stearate. Preferably, said lubricant is present in between 0.25 and 1% w/w by weight.

Flavoring agents and flavor enhancers make the dosage form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol and tartaric acid.

Solid compositions may also be dyed using any pharmaceutically acceptable colorant to improve their appearance and/or facilitate, patient identification of the product and unit dosage level.

The tablet according to the current invention is particularly suitable for use in the following medical indications and uses:
- for use in the treatment of sleep problems chosen from delayed sleep phase disorders (DSPS), primary insomnia for adults aged 55+ and general sleep disorders;
- for use in the treatment of difficulties in sleep initiation and maintenance that are not associated with a medical condition, substance use or concurrent psychological disorder;
- for use in the treatment of insomnia (sleep initiation, sleep duration and sleep efficiency) that are not associated with a medical condition, substance use or concurrent psychological disorder in adults;
- for use in the treatment of ADHD, insomnia due to ADHD, concentration and/or attention problems and/or hyperactivity;
- for use in the treatment of insomnia due to certain high blood pressure medications called beta-blockers;
- for use against sleep problems in children with developmental disorders including autism, cerebral palsy, and intellectual disabilities;
- for use as a sleep aid after discontinuing the use of benzodiazepine drugs;
- for reducing the side effects of stopping smoking;
- for use in the treatment of the symptoms of Alzheimer's disease;
- for use in the treatment of depression, chronic fatigue syndrome (CFS), fibromyalgia, migraine and other headaches, irritable bowel syndrome (IBS), bone loss (osteoporosis), a movement disorder called tardive dyskinesia (TD), epilepsy, as an anti-aging agent;
- for use in the treatment of the symptoms of menopause;
- for lowering the side effects of cancer treatment (chemotherapy) including weight loss, nerve pain, weakness, and a lowered number of clot-forming cells (thrombocytopenia);
- for use in the calming of people before they are given anesthesia for surgery;
- other uses included macular degeneration, glaucoma, protection of the gastric mucosa, irritable bowel syndrome, arterial hypertension, diabetes, side effects of chemotherapy and radiation in cancer patients or hemodialysis in patients with renal insufficiency.

By preference, the daily dose for the uses as given above will be between 1 to 10 mg, more preferably between 1 and 5 mg, more preferably between 1 and 3 mg, by preference once daily, for instance shortly before bedtime.

The invention is further described by the following non-limiting examples which further illustrate the invention, and are not intended to, nor should they be interpreted to, limit the scope of the invention.

### EXAMPLES

### Example 1: Establishment of a manufacturing process of melatonin formulations

An experiment was set up to identify the most optimal manufacturing process for melatonin formulations, offering good uniformity of dosage units results.

The following protocols were used:
P1: direct compression of the ingredients
P2: addition of a disintegrant to the composition used in P1; direct compression P3: wet granulation including binder and a disintegrant
P4: wet granulation
P5: wet granulation using ethanol as solvent
P6: wet granulation using acetone as solvent. Melatonin and binder are dissolved in acetone.
P7: wet granulation using water as solvent; the binder being dissolved in water. P8: wet granulation using a mixture of ethanol and water.
P9: dispersion of melatonin with part of the excipients by using a knife mill. One part of the mixture was used in a direct compression process (P9a), a second part was used in a wet granulation process using water as solvent (P9b).
P10: similar set up as P9, but with higher melatonin content.

The compositions used are shown in Table 1.

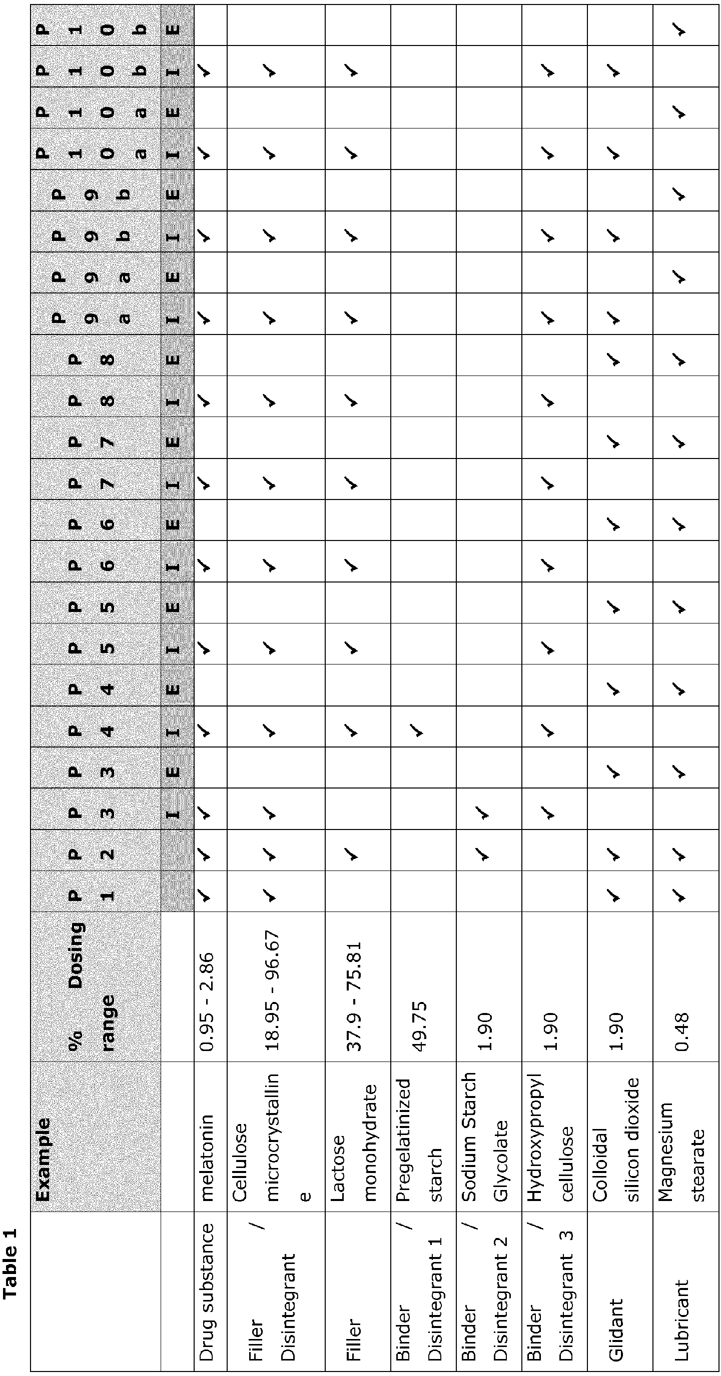

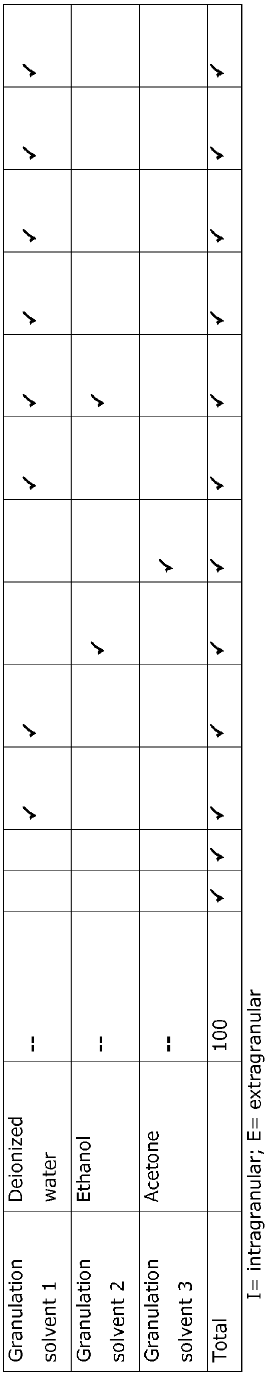

The different manufacturing protocols as described above were performed and the resulting formulation or tablet was tested for its drug liberation properties and uniformity of dosage units.

The following results were achieved (Table 2 and 3). Uniformity of Dosage Units test is executed according to the European Pharmacopoeia monograph 2.9.40. Compliance is demonstrated by measuring the Content Uniformity. To that end a state of the art High Pressure Liquid Chromatography method was developed. The requirements of this method comprise of a C18 column (150/4.6mm- 5 µm), flow 1ml/min, isocratic mobile phase composed of acetonitrile phosphate buffer (25/75), whereby the run is at a constant ambient temperature. The injection volume is 10 µl. Compound detection is carried out at 222nm. Validation of analytical method for the identification and the assay of melatonin in Melatonin tablets was carried out according to a written protocol following the ICH Q2 (R1) guideline (Validation and analytical procedures: text and methodology). Validation was carried out in a high resolution liquid chromatograph, according to Good Laboratory Practices. The method was verified to be selective for the active substance melatonin, as well as linear in the range established, precise and accurate.

**Table 2**

| Batch | **Average hardness (N)** | **Average disintegration (s)** | **Friability (%)** |
|---|---|---|---|
| P1 | 65.20 | 5.00 | 0.09 |
| P2 | 26.60 | 24.67 | 0.43 |
| P3 | 66.20 | 4.00 | 0.08 |
| P4 | 19.00 | 21.33 | 0.07 |
| P5 | 52.00 | 10 | 0.09 |
| P6 | 50.50 | 10 | 0.00 |
| P7 | 66.75 | 15 | 0.00 |
| P8 | 64.75 | 12 | 0.00 |
| P9a | 59.00 | 9.00 | - |
| P9b | 60.25 | 10.00 | - |
| P10a | 48.00 | 10.33 | 0.24 |
| P10b | 49.67 | 9.67 | 0.33 |

**Table 3 Uniformity of Dosage Units**

| | P1 | P2 | P3 | P4 | P5 | P6 | P7 | P8 | P9a | P9b | P10a | P10b |
|---|---|---|---|---|---|---|---|---|---|---|---|---|
| Average (%) (*) | 92.0 | 91.6 | 116.4 | 107 .0 | 105.16 | 103.03 | 107.51 | 113.9 | 91.3 | 102.5 | 92.8 | 100.8 |
| RSD (%) | 10.6 | 12.9 | 6.6 | 5.7 | 2.27 | 2.35 | 5.87 | 5.56 | 1.36 | 1.48 | 1.65 | 1.06 |
| AV (**) | 29.9 | 35.3 | 33.3 | 20.3 | 9.4 | 7.3 | 21.2 | 27.6 | 10.2 | 4.6 | 9.3 | 2.6 |

| | | | | | | | | | | | | |
|---|---|---|---|---|---|---|---|---|---|---|---|---|
| (*) Active ingredient as a % of the label claim=100% (**) Acceptance Value, as in accordance with Ph.Eur. 2.9.40 | | | | | | | | | | | | |

To summarize, none of the batches of P1 to P4 and P7 to P8 showed satisfying results with regard to the uniformity of dosage units (having AV values above 15). P5 and P6 pass the test. The results from our formulation experiments show that there is a relationship between the dissolution of melatonin and the uniformity of dosage units. An optimal formulation is obtained by dissolving melatonin in acetone, which completely dissolves and optimally disperses the melatonin. However, use of acetone or ethanol is strongly discouraged in the framework of industrial production of pharmaceutical formulations, due to safety risks.

P9a, P9b and P10a and 10b comply with the uniformity of dosage units test. The AV value is less than 15. Good dissolution profiles were observed (data not shown). Within 15 minutes, more than 85% of the drug substance was dissolved.

The results show that dispersing melatonin gives the best results and is the most desired process to implement in a large manufacturing plant. However, direct compression (P9a, P10a) presents a lower assay value than wet granulation process. The difference could not be justified with API weighing mistakes or API loss during the dispersion process because both were based on the same premix. Additional experiments have demonstrated that the latter was inherent to the process of direct compression and was not due to e.g. unwanted drug adsorption to plastic surfaces (data not shown).

Therefore, in the exclusion of the use of organic solvents, only melatonin dispersion, combined with a wet granulation step is shown to give the desired results.

### Example 2: Use of the formulation against sleep problems

An analysis was conducted in order to analyze the effects of a formulation according to the current invention on sleep and insomnia (1 mg melatonin, taken 1 hour before bedtime). 284 subjects were included, the study was a double-blind, randomized, placebo-controlled trial. Objective measures of sleep evaluation were used. The analysis showed that the formulation was effective considering the three objective criteria proposed by the EMA for the evaluation hypnotic drugs. Consequently, the formulation is useful in treating insomnia, particularly in aged individuals with nocturnal melatonin deficiency or with an abnormal pattern of melatonin secretion.

### Example 3: Use of the formulation against ADHD

A total of 100 medication-free children, ages 6 to 12 years, with rigorously diagnosed ADHD and chronic sleep onset insomnia participated in a randomized, double-blind, placebo-controlled trial using 3 or 6 mg melatonin (depending on body weight) with a tablet according to the current invention, or placebo for 4 weeks. Primary outcome parameters were actigraphy-derived sleep onset, total time asleep, and salivary dim light melatonin onset.

Melatonin was shown to advance circadian rhythms of sleep-wake and endogenous melatonin and enhanced total time asleep in children with ADHD and chronic sleep onset insomnia. Sleep onset generally advanced by 30 minutes to 1 hour in the melatonin treated population, compared to a delay of 10 to 40 minutes with placebo. Further, an effect on problem behavior, cognitive performance, or quality of life was observed.

### Example 4: Manufacturing of a melatonin formulation according to the current invention

Figure 1 shows a flowchart of a manufacturing process according to an embodiment of the current invention.

A possible protocol according to the current invention is as follows:
4.1 API dispersion process
   - Mix Melatonin, Aerosil and a portion of Microcrystalline Cellulose (Ratio: 1/0.2/5).
   - Sieve the blend through an appropriate siever.
4.2 Wet Granulation
   - Prepare a binder solution of Hydroxypropylcellulose in Deionized water.
   - Mix the remaining intragranular excipients (remaining microcrystalline cellulose and lactose monohydrate) with the API dispersion.
   - Knead the blend during specified time.
   - Dry the granulate until LOD <4% (by sue of a fluid bed dryer).
   - Sieve the granulate.
   - Calculate granulation process yield.
   - Add the extragranular excipients (magnesium stearate) to the granulate.
   - Mix the final blend untilhomogeneity.
4.3 Tableting process
   - Press the product using biconcave 7mm tools, obtaining tablets with the following characteristics:
      - Hardness: 40-80N
      - Friability ≤ 0.5 %
      - Disintegration ≤ 180 seconds

### 4.4 Tests

The concentration relative standard deviation of all batches was found to be not more than 2%, which lead to conclude that the homogeneity of the product was excellent.

Results of blend uniformity testing showed that the active ingredient, which is extremely fine, was properly bound and dispersed, showing that granulation and blending was successful.

In general, the obtained tablets passed all quality controls.

### Example 5: Formulation according to the current invention

Table 4 gives examples of possible formulation according to the current invention, whereby the amount of active ingredient and excipients is given in relative amounts, relative in view of the cellulose.

**Table 4**

| **Component** | **Function** | **Relative content per tablet** |
|---|---|---|
| Melatonin | Drug substance | 1.67 |
| Microcrystalline cellulose | Filler / Disintegrant | 100 |
| Lactose Monohydrate | Filler | 68.89 |
| Colloidal anhydrous silica | Glidant | 0.33 |
| Hydroxypropylcellulose | Binder Disintegrant | 3.33 |
| Magnesium stearate | Lubricant | 0.83 |
| Purified Water* | Granulation process diluent | - |

| | | |
|---|---|---|
| (*) Not present in finished product. | | |

## Claims

1. A method for the manufacturing of a solid formulation comprising melatonin as active pharmaceutical ingredient, said method comprises a melatonin dispersion step in one or more intragranular excipients to form a melatonin blend, followed by a wet granulation step.

2. Method according to claim 1, whereby said intragranular excipients in the dispersion step are chosen from fillers, binders, disintegrants, and/or glidants.

3. Method according to any one of the claims 1 or 2, **characterized in that** a binder solution is granulated with said melatonin blend.

4. Method according to claim 3, **characterized in that** said wet granulation occurs by means of a high shear granulator, fluidized bed granulator or twin screw granulator.

5. Method according to any one of the previous claims, **characterized in that** said dispersion step comprises the formation of a premix, said premix comprises melatonin and one or more intragranular excipients, followed by a sieving step of said premix and further blending with one or more intragranular excipients to form said melatonin blend.

6. Method according to claim 5, **characterized in that** said sieving is performed by use of a mesh with mesh size between 800 and 1200 micron.

7. Method according to claim 6, comprising the following sequence of steps:
- dispersion of melatonin with one or more fillers and a glidant;
- providing a binder solution;
- wet granulation of said melatonin blend with said binder solution;
- drying the obtained granulate;
- addition of a lubricant to said granulate; and mixing of the final blend.

8. Method according to claim 7, whereby said final blend is tableted.

9. Method according to claim 7 or 8, **characterized in that** said ratio between melatonin, glidant and filler is between 1:0.1:4 and 1:0.3:6.

10. Tablet comprising melatonin, whereby said tablet is an immediate release formulation and whereby said tablet comprises:
- between 0.5 and 10% w/w of melatonin;
- between 75 and 99% w/w of one or more fillers;
- between 0.5 and 5% w/w of one or more binders;
- between 0.05 and 0.5% of one or more glidants; and
- between 0.25 and 1% w/w of one or more lubricants.

11. Tablet according to claim 10, **characterized in that** said filler is selected from cellulose microcrystalline, lactose monohydrate, sucrose, glucose, polyols, calcium carbonate, and/or magnesium stearate, mixtures or derivatives thereof.

12. Tablet according to any one of the previous claims, whereby said binder is selected from cellulose or modified cellulose such as hydroxycellulose, cellulose; polyethylene glycol; polyvinylpyrrolidone; starch and/or mixtures thereof.

13. Tablet according to any one of the previous claims, whereby said glidant is selected from (colloidal) silicon dioxide, starch, talc, magnesium stearate and/or a mixture thereof.

14. Tablet according to any one of the previous claims, for use in the treatment of sleep problems chosen from delayed sleep phase disorders, primary insomnia for adults aged 55+ and general sleep disorders and treatment of difficulties in sleep initiation and maintenance that are not associated with a medical condition, substance use or concurrent psychological disorder.

15. Tablet according to any one of the previous embodiments 10 to 13, for use in the treatment of ADHD, concentration and/or attention problems and/or hyperactivity.
